# EUROPEAN PATENT APPLICATION

(11) **EP 0 962 449 A2**
(43) Date of publication of application: **08.12.1999**
(21) Application number: 99304387.6
(22) Date of filing: 04.06.1999
(51) Int. Cl.: C07C 227/22

(54) **Method for producing 3-amino-2,2,3-trimethylbutanoate**

(30) Priority: 05.06.1998 JP 15806698
(71) Applicant: Sumitomo Chemical Company, Limited, Chuo-ku Osaka 541-8550 (JP)
(72) Inventor: Ogawa, Takeshi, Narashino-shi, Chiba (JP); Haga, Toru, Niihama-shi, Ehime (JP); Imi, Katsuharu, Niihama-shi, Ehime (JP)
(74) Representative: Cresswell, Thomas Anthony

(57) **Abstract**

The present invention provides a method for producing 3-amino-2,2,3trimethylbutanoate which comprises reacting 3,3,4,4-tetramethylazetidine-2-one with an alcohol in the presence of an acidic compound, which has excellent operability and high yield.

## Description

The present invention relates to a method for producing a 3-amino-2,2,3-trimethylbutanoate. More particularly, the present invention relates to a method for producing a 3-amino-2,2,3-trimethylbutanoate from 3,3,4,4-tetramethylazetidine-2-one.

3-amino-2,2,3-trimethylbutanoate are utilized as various organic products such as medicines, agricultural chemicals and the like, or intermediates thereof.

Several methods are known for producing a 3-amino-2,2,3-trimethylbutanoate. There are, for example, a method in which alcohol and hydrogen chloride are allowed to react on a 3-(alkoxysulfonylamino)-trimethylbutanoate [Justus Liebigs Ann. Chem., vol. 661, pp. 111 to 157 (1963)], a method in which hydrochloric acid is allowed to react on an isocyanate compound of a 3-amino-2,2,3-trimethylbutanoate, and a method in which hydrogen bromide is allowed to react on a 3-(benzyloxycarbonylamino)-2,2,3-trimethylbutanoate in acetic acid[J. Chem. Soc. (C), pp. 1665 to 1666 (1971)].

However, these conventional methods are not satisfactory in the standpoints of difficulty of preparing raw materials, operability of the reaction and treatment of reaction mixture after completion of the reaction(hereinafter, referred to as post-treatment), yield of product, and the like, and is not suitable for industrial production.

The present inventors have intensively studied a method for producing 3-amino-2,2,3-trimethylbutanoate in high yield which has excellent operability, and as a result, found that the above-described object can be attained by selecting 3,3,4,4-tetramethylazetidine-2-one as a raw material and by allowing alcohol to react on this compound in the presence of an acidic compound, completing the present invention.

An object of the present invention is to provide a method for producing 3-amino-2,2,3-trimethylbutanoate which has excellent operability and high yield.

Namely, the present invention is a method for producing a 3-amino-2,2,3-trimethylbutanoate (3-amino-2,2,3-trimethylbutanoate herein referred also includes salts with acidic compounds), which comprises reacting 3,3,4,4-tetramethylazetidine-2-one with alcohol in the presence of an acidic compound.

The present invention will be further described in detail below.

The method for producing 3,3,4,4-tetramethylazetidine-2-one used as a raw material in the present invention is not particularly restricted, and it can be produced, for example, by de-chlorosulfonylation of 1-chlorosulfonyl-3,3,4,4-tetramethylazetidine-2-one. This method is, for example, disclosed in J. Org. Chem., Vol.33, No.9, 3448-3452(1968). Purity, kinds of impurities, content and the like of 3,3,4,4-tetramethylazetidine-2-one are not particularly restricted, and usually, those having a purity of 80% by weight or more, preferably about 90% by weight or more, more preferably about 95% by weight or more are used.

The alcohol used in the present invention is selected according to the intended ester, and examples thereof include primary and secondary alcohols such as methanol, ethanol, propanol, butanol, isopropyl alcohol, isobutyl alcohol, sec-butyl alcohol and the like. Among them, when methanol is used, reactivity is high and post-treatment is also easy.

Production, purity, kinds of impurities, content and the like of the alcohol used are not particularly restricted, and usually, those having a purity of about 90% by weight or more, preferably about 95% by weight or more are used. The amount used thereof is usually about 1 mol equivalent to about 50 mol equivalent, preferably about 5 mol equivalent to about 20 mol equivalent based 3,3,4,4-tetramethylazetidine-2-one.

The acidic compound used in the present invention is not particularly restricted, and examples thereof include protonic acids such as hydrogen chloride, hydrogen bromide, hydrogen iodide, sulfuric acid, nitric acid, phosphoric acid, lithium hydrogen sulfate, sodium hydrogen sulfate, potassium hydrogen sulfate, lithium dihydrogen phosphate, sodium dihydrogen phosphate, potassium dihydrogen phosphate and the like. Further, ion-exchange resins and perfluorocarbons having a sulfonic acid group and the like can also be used.

From the standpoints of reactivity and operability of post-treatment, hydrogen chloride is preferably used. Production method, purity, kinds of impurities, content and the like of the acidic compound used are not particularly restricted, and usually, those having a purity of about 90% by weight or more, preferably about 95% by weight or more are used.

It is preferable that the water content of the acidic compound used in the present invention is as low as possible, however, a certain amount of water may be contained. Water contained may undesirably produce impurities such as a carboxylic acid and the like, therefore, those having a water content of about 10% by weight or less, preferably about 5% by weight or less, further preferably about 2% by weight or less are used.

The amount used of the acidic compound is not particularly restricted, and the amount in terms of proton based on 3,3,4,4-tetramethylazetidine-2-one is usually about 1 mol equivalent to about 30 mol equivalent, preferably about 1 mol equivalent to about 15 mol equivalent, further preferably about 1 mol equivalent to about 5 mol equivalent.

A method for feeding 3,3,4,4-tetramethylazetidine-2-one, alcohol and an acidic compound in the present invention is not particularly restricted, and various methods can be adopted. For example, an acidic compound may be fed into a mixture of 3,3,4,4-tetramethylazetidine-2-one and alcohol, 3,3,4,4-tetramethylazetidine-2-one may be fed into a mixture of alcohol and an acidic compound, or 3,3,4,4-tetramethylazetidine-2-one and an acidic compound may simultaneously fed into alcohol.

Also, in the feeding, at least one of 3,3,4,4-tetramethylazetidine-2-one, alcohol and an acidic compound may be dissolved or suspended by dilution with a suitable diluent, and the solution or suspension may be fed.

Regarding 3,3,4,4-tetramethylazetidine-2-one, the solutions such as a reaction mixture in producing 3,3,4,4-tetramethylazetidine-2-one or a solution which is obtained by separation of an acidic compound and/or diluent from the reaction mixture in producing 3,3,4,4-tetramethylazetidine-2-one and the like may also be fed.

Examples of the diluent include aliphatic hydrocarbons such as hexane, cyclohexane, heptane and the like, aromatic hydrocarbons such as benzene, toluene, ethylbenzene, xylene, mesitylene and the like, ethers such as diethyl ether, diisopropyl ether, dibutyl ether, tert-butyl methyl ether, tetrahydrofuran and the like, ketones such as acetone, ethyl methyl ketone, isobutyl methyl ketone, cyclohexanone and the like, halogenated hydrocarbons such as dichloromethane, chloroform, carbon tetrachloride, dichloroethane, chlorobenzene, dichlorobenzene and the like, esters such as methyl acetate, ethyl acetate, isopropyl acetate, butyl acetate and the like, as well as other solvents, and from the standpoints of operability and property of suppressing side reaction, aromatic hydrocarbons are preferably used. The amount used of the diluent for each of 3,3,4,4-tetramethylazetidine-2-one, alcohol and acidic compound is usually from about 1 to about 20 parts by weight, preferably from about 1 to about 5 parts by weight per 1 part by weight thereof.

The reaction conditions in the present invention preferably retain the reaction system at liquid phase, and the reaction temperature is usually from about 0°C to about 150°C, preferably from about 20°C to about 80°C. The reaction pressure is preferably from about 101 kPa to about 507 kPa (about 1 atm to about 5 atm), preferably from about 101 kPa to about 203 kPa (about 1 atm to about 2 atm). The reaction time is usually from about 1 hour to about 20 hours, preferably from about 2 hours to about 10 hours. The atmosphere of the reaction system is not particularly restricted, and the reaction is preferably conducted under atmosphere of an inert gas such as nitrogen and the like.

In the present invention, since a 3-amino-2,2,3-trimethylbutanoate is a compound having an amino group, it exists in the form of a salt with an acidic compound in the reaction mixture after completion of the reaction. The post-treatment is not particularly restricted, and there can be adopted the treatment operations used in usual known organic synthesis reactions, specifically, the treatment operations used in producing known amino acid esters or salts thereof with an acidic compound.

When a 3-amino-2,2,3-trimethylbutanoate is obtained as it is (in the form of free amino group), for example, it is also permissible that to a reaction mixture or concentrated solution thereof is added a basic compound or solution thereof in an amount which can neutralize an acidic compound or more, to make the solution alkaline, then, the 3-amino-2,2,3-trimethylbutanoate is extracted with an organic solvent and the extracted solution is concentrated.

Examples of the organic solvent used include aliphatic hydrocarbons such as hexane, cyclohexane, heptane and the like, aromatic hydrocarbons such as benzene, toluene, ethylbenzene, xylene, mesitylene and the like, ethers such as diethyl ether, diisopropyl ether, dibutyl ether, tert-butyl methyl ether, tetrahydrofuran and the like, ketones such as acetone, ethyl methyl ketone, isobutyl methyl ketone, cyclohexanone and the like, halogenated hydrocarbons such as dichloromethane, chloroform, carbon tetrachloride, dichloroethane, chlorobenzene, dichlorobenzene and the like, esters such as methyl acetate, ethyl acetate, isopropyl acetate, butyl acetate and the like, as well as other solvents, and from the standpoints of operability and property of suppressing side reaction, aromatic hydrocarbon are preferably used. The amount used of the organic solvent is usually from about 1 parts by weight to about 20 parts by weight, preferably from about 2 parts by weight to about 10 parts by weight per 1 part by weight of the 3-amino-2,2,3-trimethylbutanoate.

Further, it may also be permissible that a 3-amino-2,2,3-trimethylbutanoate is once obtained in the form of a salt with an acidic compound according to the following method, then, a 3-amino-2,2,3-trimethylbutanoate is obtained according to the above-described method. In necessary, purification operations such as distillation and the like may be conducted.

As preferable operation from the standpoints of yield, product quality, operability and the like in extracting 3-amino-2,2,3-trimethylbutanoate, there is a method in which water or a basic compound or a solution thereof is added to a reaction mixture, pH is controlled preferably to about 3 to about 7, the mixture is partially concentrated, then, required amount of a basic compound or solution thereof is added to make the mixture alkaline, and a 3-amino-2,2,3-trimethylbutanoate is extracted with an organic solvent. By conducting this operation, all or most of excess alcohol and a compound having low boiling point existing in the reaction mixture (for example, in the case of use of methanol as the alcohol and hydrogen chloride as the acidic compound, methyl chloride and the like produced by reaction thereof) can be eliminated before extraction with an organic solvent, and extraction efficiency can be improved and quality degradation can be suppressed.

When a 3-amino-2,2,3-trimethylbutanoate is obtained in the form of a salt with an acidic compound, for example, there are methods as follows;
(1) A salt of a 3-amino-2,2,3-trimethylbutanoate with an acidic compound may be crystallized from the reaction mixture concentrated as it is or from the reaction mixture or concentrated solution thereof by cooling and the like, and then the deposited salt of a 3-amino-2,2,3-trimethylbutanoate with an acidic compound is separated by filtration.
(2) Components having higher ability to dissolve a salt of a 3-amino-2,2,3-trimethylbutanoate with an acidic compound such as alcohol and the like is distilled off after or while adding an organic solvent having lower ability to dissolve a salt of a 3-amino-2,2,3-trimethylbutanoate with an acidic compound to the reaction mixture or concentrated solution thereof, and then the deposited salt of a 3-amino-2,2,3-trimethylbutanoate with an acidic compound is separated by filtration.

The kind of the organic solvent used is not particularly restricted, and aromatic hydrocarbons such as benzene, toluene, ethylbenzene, xylene, mesitylene, chlorobenzene, dichlorobenzene and the like are preferably used. If necessary, purification operations such as crystallization, washing and the like may be conducted.

According to the method of the present invention, a 3-amino-2,2,3-trimethylbutanoate can be produced in high yield with excellent operability, and industrial utility thereof is extremely large.

### EXAMPLE

The following examples illustrated the present invention but do not limit the scope thereof.

The obtained product was analyzed by gas chromatography and/or liquid chromatography.

### Example 1

A round-bottom flask equipped with a stirrer, cooling tube, thermometer, dropping funnel and gas introducing tube was used, and 10 g (0.0786 mol) of 3,3,4,4-tetramethylazetidine-2-one and 40 g of methanol were fed into this flask and stirred to mix, to this was added 31 g (0,850 mol) of hydrogen chloride gas at room temperature, then, the mixture was heated under reflux for 7 hours. The reaction mixture was left for cooling, then, dried by concentration to obtain 15 g (purity;95%, 0.0728 mol, yield;93%) of methyl 3-amino-2,2,3-trimethylbutanoate hydrochloride.

### Example 2

The same apparatus as in Example 1 was used, and 10 g (0.0786 mol) of 3,3,4,4-tetramethylazetidine-2-one and 40 g of methanol were fed into the flask and stirred to mix, to this was added 37 g (1.01 mol) of hydrogen chloride gas at room temperature, then, the mixture was heated under reflux for 7 hours. 43 g of toluene was dropped to the reaction mixture with heating while the volatile component was distilled off so that the volume of the mixture was kept constant. The solution was cooled to 5°C, then, filtered, the residue was washed with 10 g of toluene then dried to obtain 11 g (purity; 99%, 0.0556 mol, yield; 71%) of methyl 3-amino-2,2,3-trimethylbutanoate hydrochloride.

### Example 3

The same apparatus as in Example 1 was used, and 55 g (0.432 mol) of 3,3,4,4-tetramethylazetidine-2-one and 220 g of methanol were fed into the flask and stirred to mix, to this was added 181 g (4.96 mol) of hydrogen chloride gas at room temperature, then, the mixture was heated under reflux for 7 hours. 302 g of toluene was dropped to the reaction mixture with heating while the volatile component was distilled off so that the volume of the mixture was kept constant. The solution was cooled to 5°C, then, filtered, the residue was washed with 100 g of toluene then dried to obtain 62 g (purity;99%, 0.314 mol, yield;73%) of methyl 3-amino-2,2,3-trimethylbutanoate hydrochloride. Further, the filtrate and the washing solution were mixed and dried by concentration to obtain 21 g (purity;95%, 0.102 mol, yield; 24%) of methyl 3-amino-2,2,3-trimethylbutanoate hydrochloride.

### Example 4

The same apparatus as in Example 1 was used, and 23 g (0.181 mol) of 3,3,4,4-tetramethylazetidine-2-one and 46 g of methanol were fed into the flask and stirred to mix, to this was added 11.5 g (0.315 mol) of hydrogen chloride gas at a temperature of 50°C over 0.5 hours, then, the mixture was kept at 50°C for 1 hour. 120 g of toluene was dropped to the reaction mixture with heating while the volatile component was distilled off so that the volume of the mixture was kept constant. The solution was cooled to 5°C, then, filtered, the residue was washed with 25 g of toluene then dried to obtain 34 g (purity;98%, 0.170 mol, yield;94%) of methyl 3-amino-2,2,3-trimethylbutanoate hydrochloride.

### Example 5

The same apparatus as in Example 1 was used, and 46 g (0.362 mol) of 3,3,4,4-tetramethylazetidine-2-one and 92 g of methanol were fed into the flask and stirred to mix, to this was added 46 g (1.262 mol) of hydrogen chloride gas with heating under reflux over 2 hours, then, the mixture was heated under reflux for 1 hour. The reaction mixture was cooled to 5°C, then filtrated, the residue was washed with 31 g of toluene, and dried to obtain 52 g (purity;99%, 0.263 mol, yield;73%) of methyl 3-amino-2,2,3-trimethylbutanoate hydrochloride. The residue contained methyl 3-amino-2,2,3-trimethylbutanoate hydrochloride in a yield of 19%.

### Example 6

The same apparatus as in Example 1 was used, and 40 g (0.314 mol) of 3,3,4,4-tetramethylazetidine-2-one and 80 g of methanol were fed into the flaask and stirred to mix, to this was added 23 g (0.631 mol) of hydrogen chloride gas with heating under reflux over 1 hour, then, the mixture was heated under reflux for 1 hour. 398 g of toluene was dropped to the reaction mixture with heating while the volatile component was distilled off so that the volume of the mixture was kept constant. After being left for cooling, 60 g of water and 50 g of 25% aqueous NaOH solution were added and mixed well, then separated into an oil layer and an aqueous layer. Further, 60 g of toluene was added to the aqueous layer and mixed well, then, separated into an oil layer and an aqueous layer. The resulted two oil layers were mixed and washed with 20 g of water to obtain 163 g of a solution containing 45 g (0.283 mol, yield;90%) of methyl 3-amino-2,2,3-trimethylbutanoate.

### Example 7

The same apparatus as in Example 1 was used, and 40 g (0.314 mol) of 3,3,4,4-tetramethylazetidine-2-one and 80 g of methanol were fed into the flask and stirred to mix, to this was added 23 g (0.631 mol) of hydrogen chloride gas with heating under reflux over 1 hour, then, the mixture was heated under reflux for 1 hour. After being left for cooling, 100 g of toluene and 50 g of 25% aqueous NaOH solution were added and mixed well, then separated into an oil layer and an aqueous layer. Further, 100 g of toluene was added to the aqueous layer and mixed well, then, separated into an oil layer and an aqueous layer. The resulted two oil layers were mixed to obtain 260 g of a solution containing 48 g (0.301 mol, yield; 96%) of methyl 3-amino-2,2,3-trimethylbutanoate.

### Example 8

The same apparatus as in Example 1 was used, and 40 g (0.314 mol) of 3,3,4,4-tetramethylazetidine-2-one and 80 g of methanol were fed into the flask and stirred to mix, to this was added 23 g (0.631 mol) of hydrogen chloride gas with heating under reflux over 1 hour, then, the mixture was heated under reflux for 1 hour. After being left for cooling, 80 g of water and 32 g of 25% aqueous NaOH solution were added to make pH 5, then, concentrated to reach a temperature of 50°C at 13.3 kPa(100 mmHg). 100 g of toluene and 88 g of 25% aqueous NaOH solution were added to the concentrated solution and mixed well, then, separated into an oil layer and an aqueous layer. Further, 100 g of toluene was added to the aqueous layer and mixed well, then, separated into an oil layer and an aqueous layer. The resulted two oil layers were mixed to obtain 243 g of a solution containing 48 g (0.301 mol, yield; 96%) of methyl 3-amino-2,2,3-trimethylbutanoate. This solution was concentrated to reach a temperature of 80°C at 13.3 kPa(100 mmHg), then, distilled to reach a temperature of 80°C at 1.33 kPa(10 mmHg), to obtain 41 g of methyl 3-amino-2,2,3-trimethylbutanoate having a purity of 99% or more.

### Example 9

The same apparatus as in Example 1 was used, and 40 g (0.314 mol) of 3,3,4,4-tetramethylazetidine-2-one and 80 g of methanol were fed into the flask and stirred to mix, to this was added 57 g (0.570 mol) of conc. sulfuric acid(98% by weight) with heating under reflux over 1 hour, then, the mixture was heated under reflux for 1 hour. After being left for cooling, 80 g of water and 64 g of 25% aqueous NaOH solution were added to make pH 7, then, concentrated to reach a temperature of 50°C at 13.3kPa(100 mmHg). 100 g of toluene and 46 g of 25% aqueous NaOH solution were added to the concentrated solution and mixed well, then, separated into an oil layer and an aqueous layer. Further, 100 g of toluene was added to the aqueous layer and mixed well, then, separated into an oil layer and an aqueous layer. The resulted two oil layers were mixed to obtain 231 g of a solution containing 36 g (0.226 mol, yield;72%) of methyl 3-amino-2,2,3-trimethylbutanoate.

### Example 10

The same apparatus as in Example 1 was used, and 10 g (0.0786 mol) of 3,3,4,4-tetramethylazetidine-2-one and 40 g of methanol were fed into this flask and stirred to mix, to this was added 84 g (0,850 mol) of conc.hydrochloric acid(37 % by weight) at room temperature, then, the mixture was heated under reflux for 7 hours. The reaction mixture was left for cooling, then, dried by concentration to obtain 16 g of mixture of methyl 3-amino-2,2,3-trimethylbutanoate hydrochloride and 3-amino-2,2,3-trimethylbutanoic acid hydrochloride. The content of methyl 3-amino-2,2,3-trimethylbutanoate hydrochloride in the mixture was 61%(Yield:61%), and the content of 3-amino-2,2,3-trimethylbutanoic acid hydrochloride in the mixture was 29%(Yield:31%).

### Comparative Example 1

The same apparatus as in Example 1 was used, and 10 g (0.0395 mol) of methyl 3-(methoxysulfonylamino)-2,2,3-trimethylbutanoate and 40 g of methanol were fed into the flask and stirred to mix, to this was added 27 g (0.741 mol) of hydrogen chloride gas at room temperature, then, the mixture was heated under reflux for 7 hour. The reaction mixture was left for cooling, then, dried by concentration to obtain 10 g (purity;15%, 0.0077 mol, yield;19%) of methyl 3-amino-2,2,3-trimethylbutanoate hydrochloride.

## Claims

1. A method for producing a 3-amino-2,2,3-trimethylbutanoate, which comprises reacting 3,3,4,4-tetramethylazetidine-2-one with an alcohol in the presence of an acidic compound.

2. A method according to claim 1, wherein said 3-amino-2,2,3-trimethylbutanoate is a salt of a 3-amino-2,2,3-trimethylbutanoate with an acidic compound.

3. A method according to claim 1 or 2, wherein the water content of said acidic compound is about 10 weight % or less.

4. A method according to any one of the preceding claims, wherein said acidic compound is hydrogen chloride.

5. A method according to any one of the preceding claims, wherein said alcohol is methanol.

6. A method according to any one of the preceding claims, wherein said reaction is conducted in the presence of a solvent.

7. A method according to any one of the preceding claims, which further comprises adding an aromatic hydrocarbon to the reaction mixture after completion of the reaction or the concentrated solution thereof, and distilling off excess alcohol.

8. A method according to any one of the preceding claims, which further comprises adding water, a basic compound or a solution thereof to the reaction mixture after the completion of the reaction, and concentrating the mixture.
